# DEMANDE DE BREVET EUROPEEN

(11) **EP 0 850 949 A2**
(43) Date de publication de la demande: **01.07.1998**
(21) Numéro de dépôt: 97117556.7
(22) Date de dépôt: 28.03.1994
(51) Int. Cl.: C07H 19/10, C12Q 1/68

(54) **Nouveaux dérivés utilisables en séquencage d'acides nucléiques**

(30) Priorité: 26.03.1993 FR 9303538
(62) Demande divisionnaire de: 94911988.7
(71) Demandeur: INSTITUT PASTEUR, 75724 Paris Cédex 15 (FR)
(72) Inventeur: Canard, Bruno, Dep. of Bio. Chem. and Molec. Pharm, Boston, MA 02115 (US); Sarfati, Simon, 75011 Paris (FR)
(74) Mandataire: Becker, Philippe

(57) **Abrégé**

La présente invention concerne des esters de désoxyribonucléotides 5' triphosphates (dNTP) diguanosine cytosine thymine ou ribonucléotides 5' triphosphates (NTP), caractérisés par le fait qu'ils sont des anthranylates répondant à la formule générale suivante : 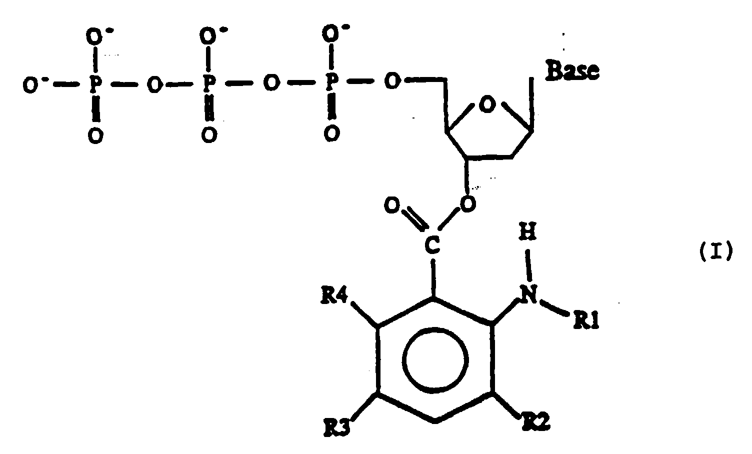
dans laquelle R1, R2, R3 ou R4 sont soit un atome d'hydrogène (H), soit un groupe méthyle (CH₃), soit un groupe autre pourvu que les propriétés du résidu soient conservées,
dans laquelle quand la base est l'adénine, au moins un des radicaux R1, R2, R3 ou R4 est différent de H,
et dans laquelle l'hydroxyle en 3' peut être restauré par libération du résidu anthranylique en milieu basique ou par action d'une enzyme, par exemple une lipase ou une hydrolase.

## Description

La présente invention est relative à des esters de désoxyribonucléotides-5' triphosphates ou ribonucléotides-5' triphosphates et leur utilisation dans une méthode de séquençage d'acides nucléiques.

La position 3' du résidu désoxyribose ou ribose est estérifiée par un dérivé particulier donnant à chaque nucléotide des propriétés fluorescentes spécifiques.

La synthèse chimique d'Ant-dATP a été décrite par Sarfati et al., J. Biol. Chem. (1990), 265, pp. 18902-18906.

Des composés voisins avec un ester fluorescent aux propriétés spectrales différentes sont décrits par Hiratsuka [1982), J. Biol. Chem., 257, pp. 13354-13358].

Ces nucléotides triphosphates estérifés (3'-RT-dNTP) sont des substrats des ADN ou ARN polymérases lesquels lorsqu'ils sont incorporés conduisent à la terminaison de la chaîne polynucléotidique.

Ils peuvent se prêter à trois réactions distinctes, à savoir l'incorporation par une acide nucléique polymérase dans une chaîne en élongation, la déprotection de l'hydroxyle en 3' du désoxyribose permettant l'incorporation d'un 3'-RT-dNTP suivant. Ces propriétés sont utilisables dans une nouvelle méthode non radioactive ne reposant pas sur l'utilisation d'un gel, pour déterminer une séquence de nucléotides ou détecter des mutations dans une séquence d'ADN.

Une des caractéristiques essentielles de ces nucléotides triphosphates estérifiés est la réversibilité in situ de cette estérification permettant la restauration des groupes 3' hydroxy libres, la chaîne polynucléotidique pouvant subir à nouveau une élongation lors d'une incubation avec un mélange de dNTP et d'ADN polymérase.

Chaque ester de nucléotide ayant des propriétés fluorescentes spécifiques d'une base donnée, il est possible, après élimination et caractérisation de ce marqueur fluorescent de déterminer laquelle des nucléobases a été insérée.

La réitération du procédé fournit ainsi une méthode de détermination d'une séquence de nucléotides ou de détection de mutations ponctuelles dans une séquence de nucléotides ou de recherche des variants ou enfin de diagnostic de la présence d'une séquence oligonucléotidique particulière dans un échantillon.

Les techniques de séquençage classique ont été introduites il y a environ 15 ans par Sanger, F., Nicklen, S. et Coulson, A.R. [(1977) Proc. Natl. Acad. Sci. USA 74, pp. 5463-5467], d'une part, et par Maxam, A.M. and Gilbert, W. [(1977) Proc. Natl. Acad. Sci. USA 74, pp. 560-564], d'autre part.

Le séquençage didésoxy de Sanger est maintenant largement utilisé et est encore une méthode de choix pour déterminer une séquence de nucléotides à partir d'une matrice d'ADN simple brin.

Brièvement, cette méthode est la suivante : pendant les 4 élongations de chaîne enzymatique, les didésoxynucléotides sont insérés de façon aléatoire à la place de leur désoxynucléotide correspondant. Les réactions de séquençage génèrent un mélange complexe qui est ensuite résolu par électrophorèse sur gel de polyacrylamide.

Cette méthode est relativement complexe quant aux étapes suivant l'incorporation des didésoxynucléotides, notamment pour ce qui est de la résolution sur gel, d'une part, et d'acquisition de données, d'autre part.

On se trouve en effet en présence d'une grande diversité de produits générés par l'élongation.

Différentes améliorations ont ensuite été apportées à cette technique avec l'objectif de simplifier et d'alléger les manipulations expérimentales qu'elle implique.

Par exemple, dans la demande de brevet EP-0 531 169 A1 les auteurs ont développé une technique permettant de simplifier et d'affiner l'étape d'électrophorèse en utilisant les techniques d'électrophorèse en champ pulsé.

D'autres perfectionnements plus récents des techniques de séquençage ont été décrits dans la littérature. Nous en citerons deux :
- la technique 〈〈 séquençage de DNA multiplexe 〉〉 [G.M. Church et S. Kieffer-Higgins, Science, 240, pp. 185-188 (1988)];
   cette technique implique la formation de génomes artificiels à partir d'un mélange de fragments insérés dans des vecteurs multiples adjacents à différents oligonucléotides marqués ; le DNA est ensuite coupé chimiquement et subit une électrophorèse, puis est transféré sur des membranes et sondé enfin avec des oligonucléotides complémentaires des séquences marquées dans le vecteur ;
- l'autre technique, bien connue, a été décrite dans O. Ohara et al. Proc. Natl. Acad. Sci USA 86, pp. 6883-6887 (1989) ;
   cette technique utilise également des systèmes de gel et de membranes permettant de repérer les fragments hybridés avec les sondes oligomériques.

Dans la demande de brevet internationale WO 93/02212, les inventeurs décrivent un procédé d'amplification et de séquençage d'ADN et d'ARN en une seule étape.

Ce procédé implique l'utilisation d'analogues de nucléotides de type désoxy conduisant à la terminaison de la chaîne et ce, de façon réitérée, la séparation des produits de la réaction étant faite ensuite sur gel de polyacrylamide.

La technologie du séquençage fait l'objet d'études intenses, principalement en raison du développement des projets sur le génome. Beaucoup d'améliorations sont orientées vers la méthode didesoxy au niveau de plusieurs de ces étapes. On pourra citer par exemple les articles de Venter, C.J. et al. [(1992) T.I.B.S 10, 8-11], de Prober, J.M. et al. [(1987) Science 238, pp. 336-341] et de Mathies, R.A. et Huang, X.C. [(1992), Nature 359, pp. 167-169].

De nouvelles approches sont en train d'apparaître, comme le séquençage par hybridation [Strezoska, Z. et al. (1991) Proc. Natl. Acad. Sci. USA 88, pp. 10089-10093] ou la microscopie à effet tunnel [Driscoll, R. J. et al. (1990) Nature 346, pp. 294-296], lesquelles méthodes peuvent se développer largement si l'électrophorèse sur gel est éliminée et remplacée par une variante simple et peu coûteuse, comme celle de l'invention.

Pour ce qui est des méthodes d'identification d'un nucléotide présent à une position définie dans un acide nucléique, la demande de brevet WO 93/02212 décrit une telle méthode d'identification par incorporation d'un didésoxynucléotide, mais cette méthode, si elle permet la détection d'une mutation ponctuelle, ne permet en aucun cas la détermination d'une séquence dans la mesure où aucune possibilité de restauration d'un nucléotide normal après incorporation du nucléotide modifié dans la chaîne en croissance n'est évoquée. De plus, elle nécessite de positionner l'amorce à côté du nucléotide d'intérêt, et ne permet pas de caractériser des mutations plus complexes comme de petites insertions ou délétions.

Enfin, les didésoxynucléotides sont de fabrication et d'utilisation coûteuses.

La demande de brevet WO 91/06678 décrit une méthode de séquençage d'ADN sans gel et des dispositifs pour mettre en oeuvre cette méthode. La réaction y implique des dNTP bloqués en 3'. Cependant, les enzymes et leur mode d'utilisation cités - notamment la séquénase - ne permettent pas l'utilisation simultanée de nucléotides estérifiés en 3', ni la mise en oeuvre du procédé lorsque l'ADN à séquencer présente des répétitions d'un nucléotide donné ; la séquénase possède en effet une activité estérase intrinsèque.

Une réaction idéale de séquençage produirait un seul produit d'addition de façon contrôlée, permettant l'identification du nucléotide ajouté, avant la répétition du procédé, conduisant à une détermination de la séquence par étape et en temps réel.

La protection de l'extrémité 3' de la molécule d'ADN en élongation, laquelle protection étant réversible, pourrait être une méthode conférant les propriétés désirées pour une telle réaction. Si, en outre, chaque nucléotide modifié à l'extrémité 3' porte un marquage individuel et distinct, on peut ainsi envisager de déterminer la séquence pas à pas, en temps réel.

L'invention consiste en la synthèse de nouveaux dérivés de dNTP ou de NTP permettant leur utilisation comme terminateurs de croissance de chaîne d'acides nucléiques, cette terminaison pouvant être rendue réversible par hydrolyse chimique ou enzymatique, notamment une estérase, étape du processus étant stable.

Ces dérivés peuvent être avantageusement utilisés dans tout procédé de séquençage tel que cité ci-dessus caractérisé en ce que le procédé utilise ou non un gel à une quelconque de ses étapes.

Les dNTP ou les NTP de l'invention sont plus particulièrement caractérisés par le fait qu'ils sont estérifiés en 3' du résidu désoxyribose ou ribose, soit par des anthranylates, soit par l'acide caproïque amidifié par 4 fluorophores différents correspondant chacun à une base, soit enfin par l'acide 5-amino-2,5-didésoxy-D-ribonique, soit par l'acide 6amino-2,3,6-tridésoxy-D-gluconique, tous deux également substitués sur la fonction amine par 4 fluorophores différents.

Ces quatre types d'esters étant représentés par les formules I, II, III et IV suivantes :

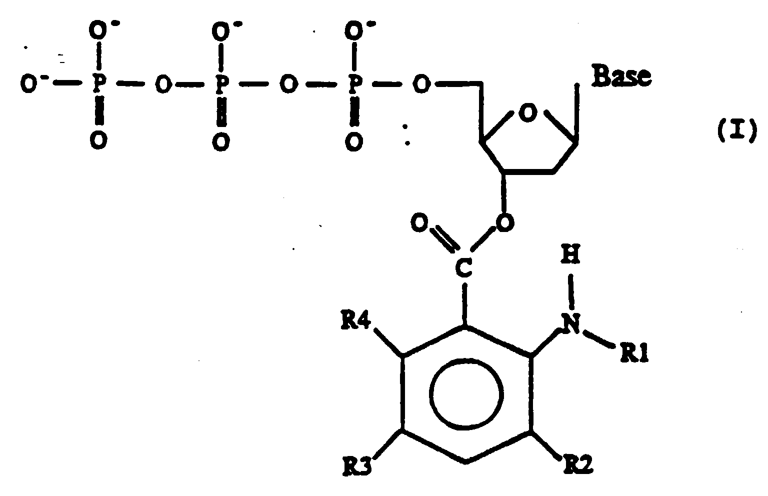

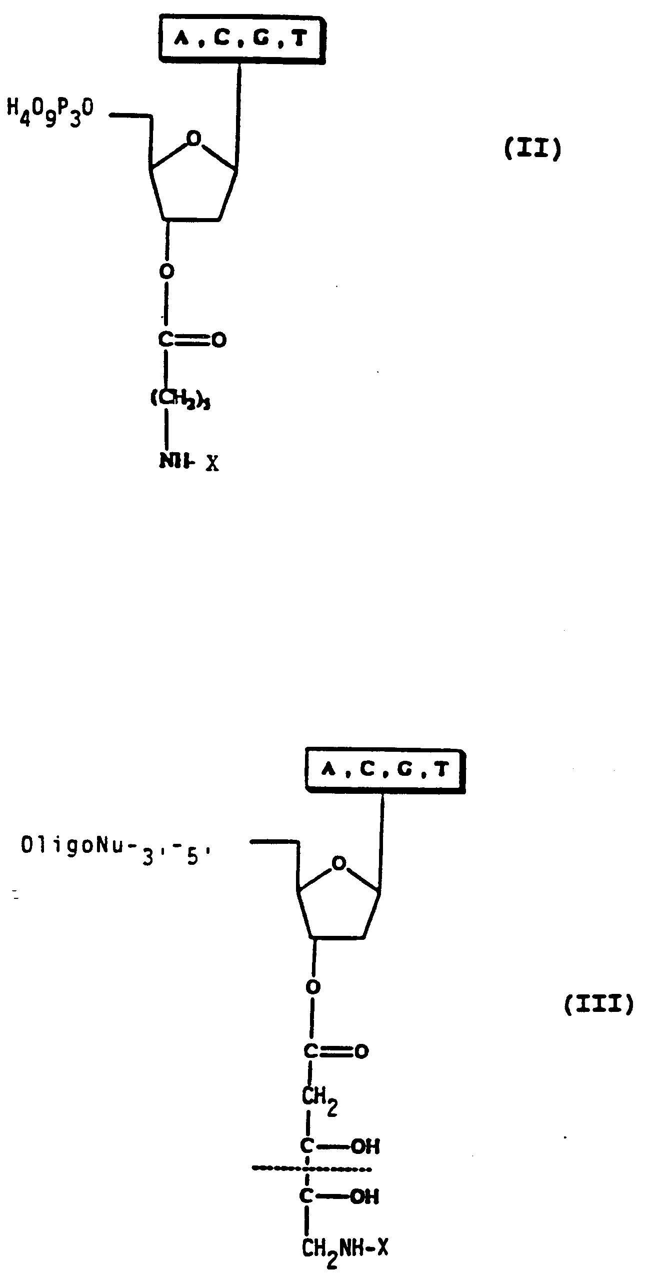

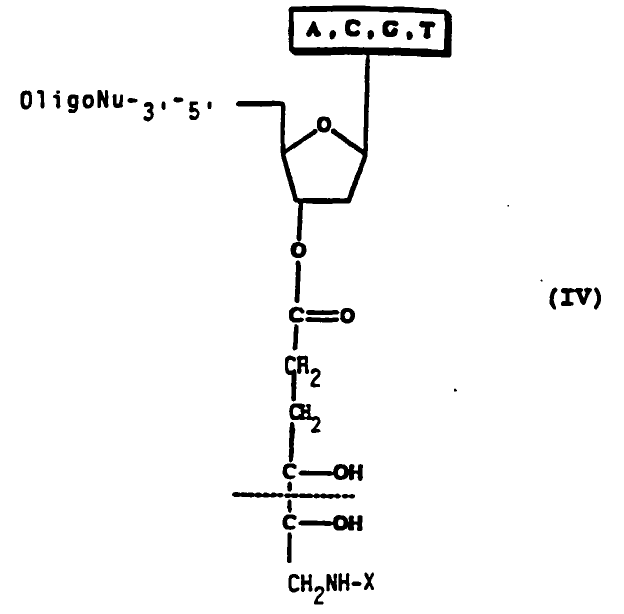

La restauration de la fonction hydroxyle en 3' du sucre peut être réalisée soit par des bases telle la soude, soit par action d'une enzyme également dans les composés de la formule I et de la formule II. Pour ce qui est des composés de formules III et IV, l'invention consiste en un clivage du fluorophore en un site éloigné de 2 ou 3 carbones de la position 3' du nucléotide.

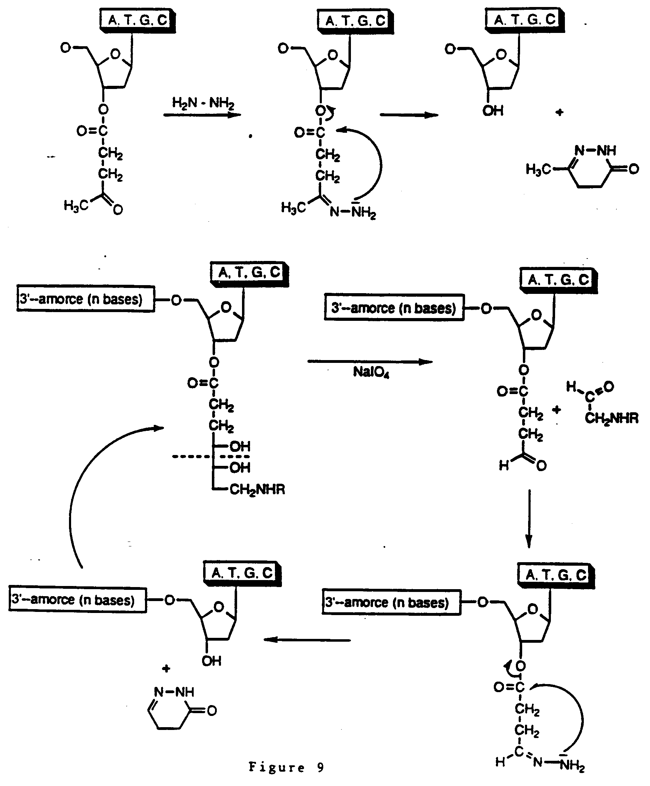

Cette réaction chimique génère des groupements réactifs qui produisent alors une réaction secondaire conduisant à la restauration de l'hydroxyle en 3', soit deux étapes qui sont :
- une oxydation du diol vicinal par le periodate,
- une énolisation, puis une β-élimination de l'aldéhyde ainsi formé en 3' pour le composé III,
- une élimination sous l'action de l'hydrazine dans le cas du composé IV (voir les détails dans la figure 9).

L'invention est également relative au procédé de synthèse de ces. différentes classes de dNTP estérifiés en 3' conduisant à la synthèse des 4 esters présentant des propriétés fluorométriques différentes.

L'invention est également relative à la construction et à l'utilisation d'une amorce en 〈〈 épingle à cheveux 〉〉 phosphorylée en 5' présentant une partie de sa séquence en 3' identique à celle d'une amorce utilisée en PCR pour produire la matrice d'ADN à séquencer (voir exemple ci-dessous).

Le terme d'amorce signifie toute séquence d'oligonucléotides qui, hybrididée avec une matrice d'acide nucléique permet à une polymérase d'initier la synthèse du brin complémentaire.

Une amorce en épingle à cheveu est liée de façon covalente par son extrémité 3' à l'extrémité 5' du brin d'acide nucléique portant la séquence recherchée.

L'utilisation de cette amorce en 〈〈 épingle à cheveux 〉〉 permet d'utiliser des conditions basiques de déprotection de l'hydroxyle en 3' compatibles avec une réitération du procédé et ce, sans rajout d'amorce à chaque étape de détermination indirecte d'un nucléotide inséré. En effet, la re-hybridation de l'amorce s'effectue intramoléculairement de façon immédiate.

Enfin, l'invention est relative à l'utilisation de ces nucléotides estérifiés dans une méthode de détermination d'une séquence de nucléotides ne reposant pas sur l'utilisation d'un gel. Ladite séquence peut être préalablement amplifiée par la technique PCR.

L'utilisation de ces nucléotides estérifiés peut également être appliquée à la détection de mutations ponctuelles ou des petites mutations type délétions ou additions, ou enfin à la recherche de variants dans une séquence génétique donnée.

Enfin, ces nucléotides triphosphates modifiés peuvent être utilisés dans une méthode de diagnostic pour détecter la présence d'une séquence oligonucléotidique particulière dans un échantillon.

Enfin, l'invention concerne une trousse de diagnostic comprenant éventuellement une amorce PCR, un amorce de séquençage, 4 2'-désoxyribonucléotides estérifiés en 3' de façon réversible, et éventuellement une phase solide pour immobiliser l'acide nucléique cible ou l'amorce, et enfin une acide nucléique polymérase choisie en fonction de l'amorce.

### DESCRIPTION DETAILLE DE L'INVENTION:

L'invention est décrite plus en détail dans la description ci-après accompagnée des figures dont la signification est la suivante :

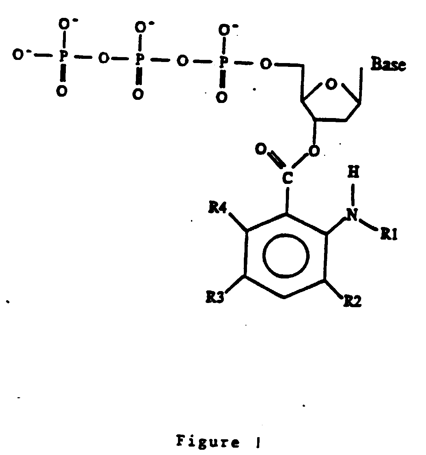

- la **Figure 1** représente la structure des esters d'anthranylates dans laquelle R1 à R4 représentent soit un groupe -H, soit un groupe -CH₃ selon le 3'-RT-dNTP considéré,
   - le 3'-Ant-dATP : R1=R2=R3=R4=H,
   - le N-méthyl-3'-Ant-dCTP : R1=CH₃ ; R2=R3=R4=H,
   - le 3-méthyl-3'-Ant-dTTP : R1=R3=R4=H; R2=CH₃ et
   - le 5-méthyl-3'-Ant-dCTP : R1=R2=R4=H; R3=CH₃.

Toute autre combinaison de groupes substituants sur le cycle anthranylique fait également partie de l'invention.
- La **Figure 2** représente la structure des esters d'acides caproïques, et une des voies de synthèse de ces esters.
- La **Figure 3** représente la formule des dérivés estérifiés en 3' par l'acide 5-amino-2-5-didésoxy D-ribonique substitué sur la fonction amine par quatre fluorophores différents, et la méthode de restauration de la fonction 3'OH du sucre par action du periodate, puis β-élimination après énolisation.
- La **Figure 4** représente la cinétique d'incorporation des dérivés 3'-Ant dATP dans une chaîne en élongation, en fonction de différentes quantités d'ADN polymérase.
- La **Figure 5** représente l'incorporation du 3'-Ant dATP en présence de différentes ADN polymérases - l'incubation est effectuée 60 minutes à 37°C avec une unité de chaque enzyme.

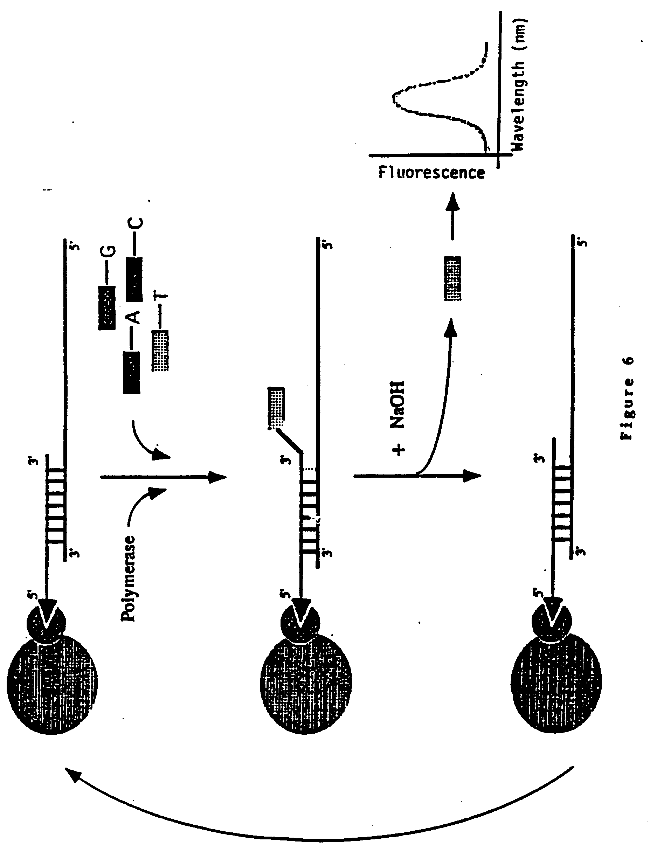

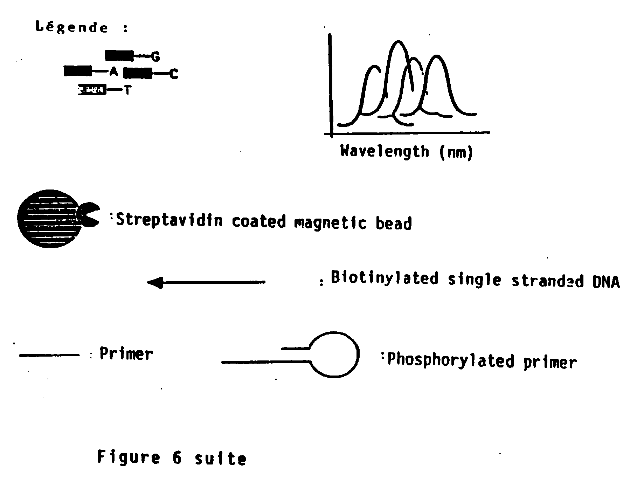

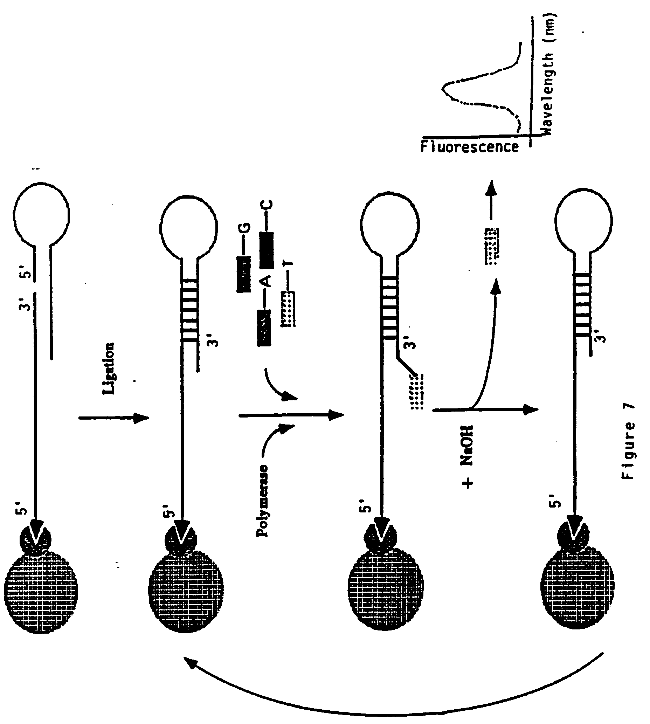

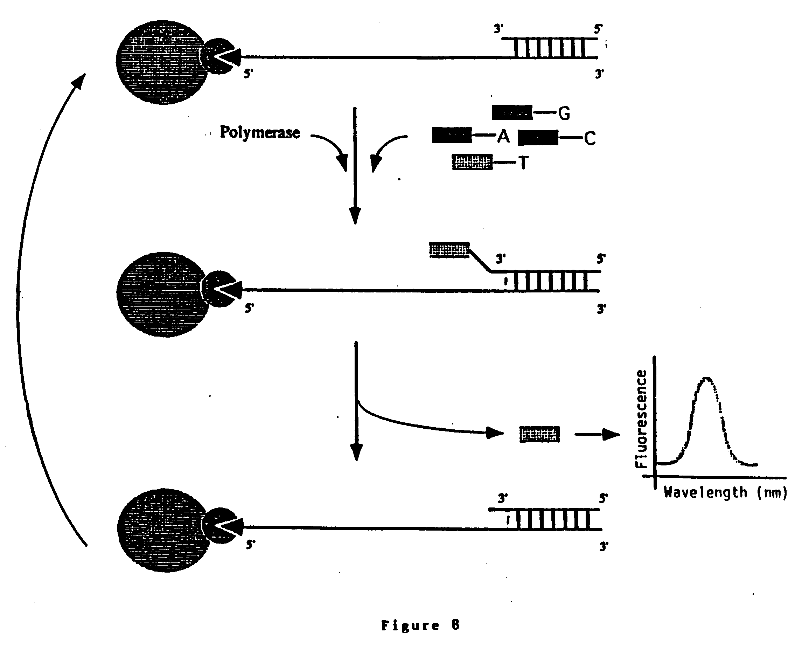

- Les **Figures 6, 7 et 8** représentent trois variantes de la méthode de séquençage utilisant les 3'-RT-dNTP de l'invention dans lesquelles les symboles ont la signification indiquée sous la Figure 6.
- La **Figure 9** représente le mécanisme de libération de l'étiquette et de l'hydroxyle en 3' des 2'-désoxynucléotides triphosphates acylés en 3' par l'acide 6-amino 2,3,6-tridésoxy-D-gluconique substitué sur la fonction amine par différents fluorochromes.
- Les **Figures 10 et 11** représentent respectivement les schémas de synthèse de la 2'-désoxyadénosine et de la 2'-désoxyguanosine triphosphates acylées en 3' par l'acide 6-amino-caproïque substitué par un fluorochrome. A la **Figure 11 suite B**, sur le côté inférieur droit, est représentée la structure du composé monophosphate constituant un intermédiaire de synthèse des esters de désoxyribonucléotides 5' triphosphates (d NTP) diguanosine cytosine thymine ou ribonucléotides 5' triphosphates (NTP) constitués par les composés anthranylates de formule (I).

Les **matériels et méthodes** utilisés sont les suivants:
- Produits pour la synthèse des dérivés :
   L'anhydride isatoïc, l'anhydride N-méthylisatoïc, l'acide 2-amino-3-méthylbenzoïc, l'acide 2-amino-5-méthyl-benzoïc et l'acide 2-amino-6-méthyl-benzoïc proviennent de chez Aldrich. Les anhydrides correspondants ont été préparés par action du phosgène ou du chloroformate d'éthyle sur les acides (Erdmann). Les nucléotides triphosphates, dATP, dGTP, dTTP et dCTP proviennent de chez Boehringer Mannheim.
- Réaction d'incorporation :
   L'ADN polymérase modifiée dépourvue d'exonucléase (Sequenase 2,0) provient de USB corp. (USA). Les 2'-3'-didéoxynucléotide / 2'-désoxynucléotide-5'-triphosphates, AMV-reverse transcriptase, M-MuLV reverse transcriptase proviennent de Boehringer (FRA). L'ADN polymérase T7 provient de Pharmacia (France), l'enzyme de Klenow provient d'Amersham (France). Une unité est l'activité enzymatique qui incorpore 1,0 nmole de nucléotides totaux dans un produit insoluble dans l'acide en 1 minute à 37°C avec du poly(d(A-T)) comme matrice.
   Les billes magrtétiques revêtues de streptavidine (M-280 Dynabeads) proviennent de Dynal (Norvège). Les α-³²P-dCTP (3000 Ci/mmole), α-³⁵S-dATP (600 Ci/mmole), ³H-dGTP (14 Ci/mmole), ³H-dCTP (17 Ci/mmole), ³H-dTTP (45 Ci/mmole) proviennent d'Amersham (France).
   Les oligonucléotides sont synthétisés avec un synthétiseur ABI et purifiés avant emploi.
- Mesures spectrales :
   Les spectres d'absorption sont enregistrés à 25°C dans un spectrophotomètre à double faisceau en présence de Tris-HCl 50 mM (pH 8,0). Les spectres d'émission de fluorescence et les spectres d'excitation sont mesurés à 25°C dans un spectrophotomètre à fluorescence Perkin-Elmer LS50B. Tous les dérivés d'acide libre sont excités à 310 nm, tandis que les dérivés 3'-anthranyloyles et 3'-méthylanthranyloyles des désoxynucléotides sont excités à 330 nm. Les largeurs de fente pour l'excitation et l'émission sont de 2,5 nm.

Les longueurs d'ondes d'absorption et d'émission pour chacun de ces dérivés dNTP anthranyloyles, comparées à celles des dérivés non substitués sont résumées dans le tableau I suivant dans lequel R1, R2, R3 et R4 correspondent aux résidus de la Figure 1.

**TABLEAU 1**

| Free Acids | | | | | | 3'-RT-dNTP | | |
|---|---|---|---|---|---|---|---|---|
| R1 | R2 | R3 | R4 | λmax* absorption | λmax* emission | dNTP | λmax* absorption | λmax* emission |
| H | H | H | H | 315 | 396.5 | dATP | 333 | 427 |
| CH₃ | H | H | H | | 416.5 | dGTP | 356 | 444 |
| H | CH₃ | H | H | 312 | 403 | dCTP | | 432 |
| H | H | CH₃ | H | 317 | 409 | dCTP | | |
| H | H | H | CH₃ | 289 | 403 | dTTP | | 435 |

### - Synthèse, purification et caractérisation des 2'-désoxynucléotide-5'-triphosphates marqués en 3' de façon réversible (3'-RT-dNTPs) :

### a) Anthranylate dATP :

L'anthranylate dATP a été préparé à partir dATP et d'anhydride isatoïque par un procédé similaire à celui décrit par Hiratsuka [T. Hiratsuka (1982) J. Biol. Chem. **257**, pp. 13354-13358] pour la synthèse de l'anthranylate ATP et a été purifié par chromatographie sur Lichroprep RP18 (25-40 µm), en utilisant 1 mM d'acétate de triéthylammonium comme éluant.

Le dATP n'ayant pas réagi est élué en premier. L'Ant-dATP est ensuite élué avec un mélange d'acétate de triéthylammonium 1 mM et d'acétonitrile (9:1 v/v). Les fractions contenant l'Ant-dATP pur sont testées en chromatographie en couche mince sur des gels de silice en utilisant comme solvant le mélange CHCl₂/MeOH/NH₄OH (65:35:10, v/v/v), puis lyophilisées.

20 mg de nucléotide pur (0.032 mol) sont obtenus à partir de 24 mg de dATP (0.04 mol), ce qui représente un rendement final de 80 %.

L'identité et la pureté du composé sont vérifiées par spectrophotométrie UV (A₂₅₃/A₃₃₃ = 4.3, ξ²⁵³mM=20), par spectrométrie de masse (FAB+) M+ 610, m/e 611, (M+H)+.

Les propriétés spectrales du produit en RMN du proton et du phosphore sont les suivantes, à 300 MHz :
(300 MHz ;D₂O) 8.44 (s', 1H, H-8), 8.11 (s, 1H, H-2), 7.83 (d, 1H, H-6A), 7.24 (t, 1H, H-4A), 6.72 (d, 1H, H-3A), 6.66 (t, 1H, H-5A), 6.48 (dd, 1H, H-1), 5.62 (dd, 1H, H-3'), 4.38 (m, 1H, H-4'), 4.19 (m, 1H, H-5'), 4.09 (m, 1H, H-5''), 2.83 (m, 1H, H-2'), 2.69 (m, 1H, H-2'').
³P (121.5 MH₂ ; D₂O)-10.02(d, Pγ), -10.84 (d, Pα), -22.48 (t, Pβ).

### b) Anthranylates de dCTP, dGTP et de dTTP :

La synthèse, la purification et la caractérisation ont été effectuées par des méthodes similaires.

Les 3-méthyl, 5-méthyl et 6-méthyl isatoïc anhydrides ont été préparés par le procédé décrit par Erdmann [E. Erdmann (1899) Berichte **3**, pp.2159-2172].

Leurs propriétés spectrales ont également été mesurées, ainsi que celles du N-méthyl Ant-dGTP, du 3-méthyl Ant-dCTP, du 6-méthyl Ant-dTTP et du 5-méthyl Ant-dCTP.

### c) Synthèse des esters de dNTP et d'acide caproïque, amidifiés par les fluorophores :

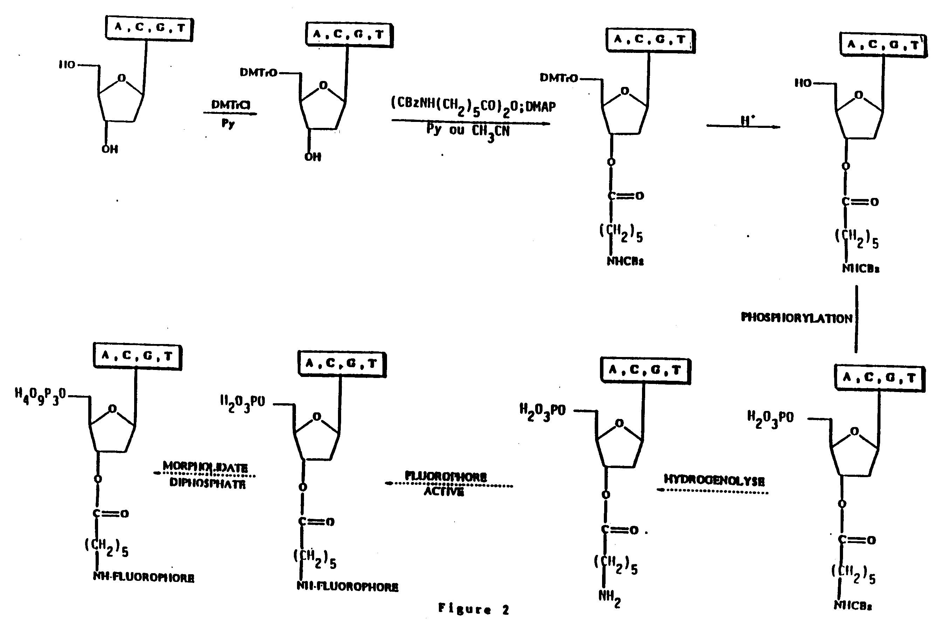

Le schéma est représenté dans la Figure 2.

L'hydroxyle en 5' du 2'-désoxy- nucléoside est protégé par un groupement diméthoxytrityle et l'alcool en position 3' est estérifié par action de l'anhydride de l'acide caproïque dont l'amine a été préalablement bloquée par un groupement benzyloxycarbonyle. L'alcool primaire en 5' est libéré en milieu faiblement acide puis phosphorylé. La fonction amine de l'ester caproïque est libérée par hydrogénolyse, puis substituée par le fluorophore.

Dans une dernière étape, le monophosphate activé sous la forme phosphoroimidazolate, est transformé en triphosphate par action du sel de tetratri-n-butylammonium de l'acide pyrophosphorique.

### d) Synthèse des purines triphosphates acylées en 3' par l'acide 6-amino-caproïque substitué par différents fluorochromes :

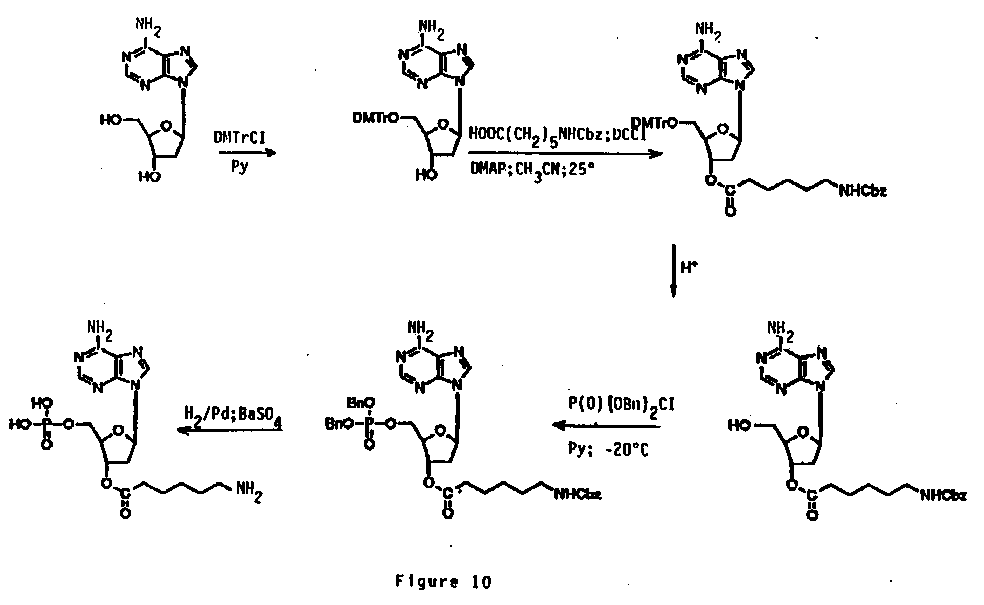

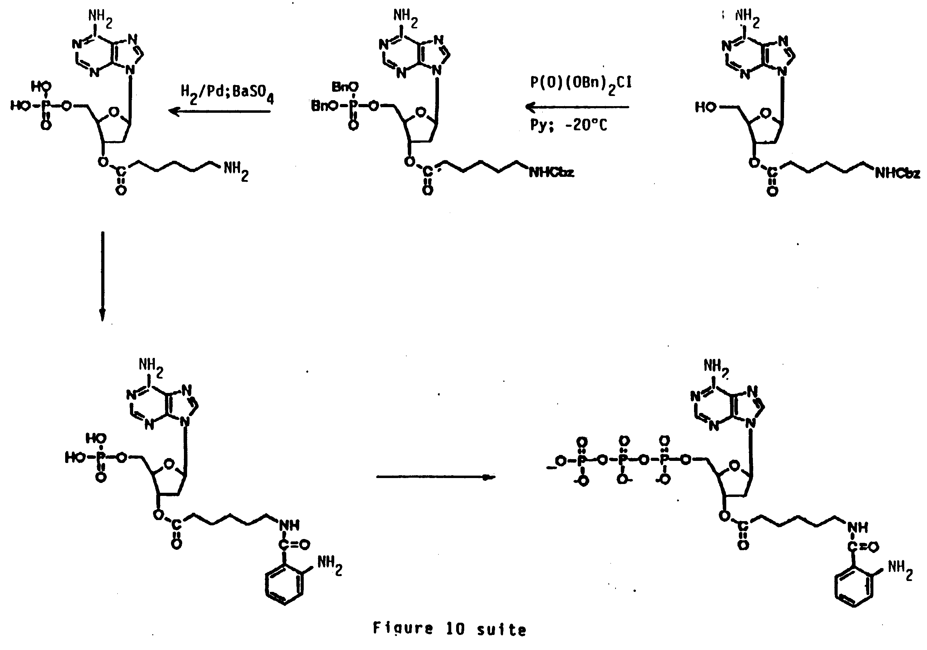

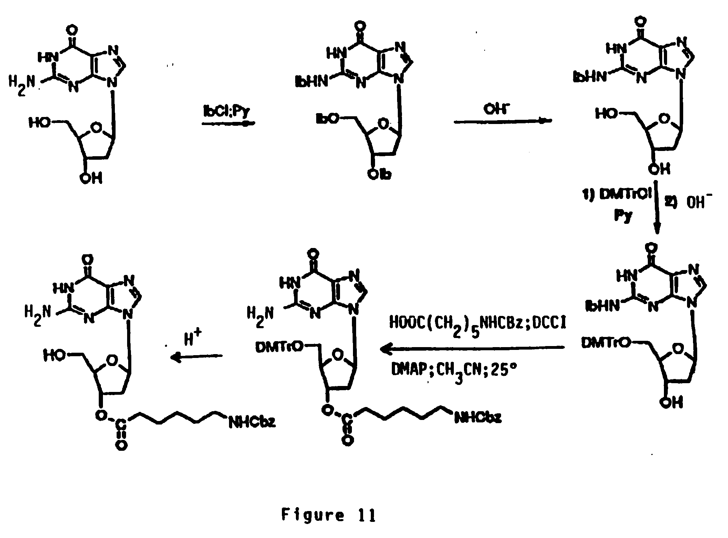

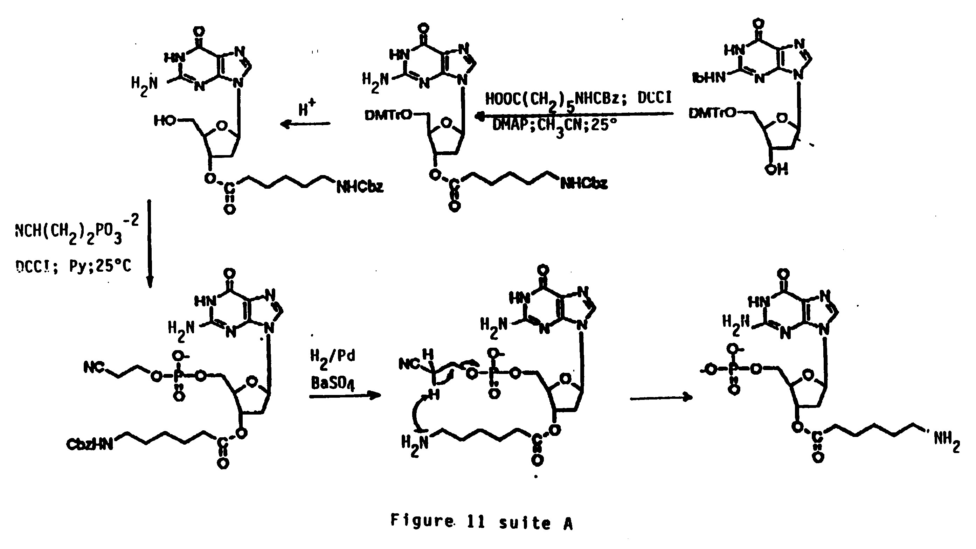

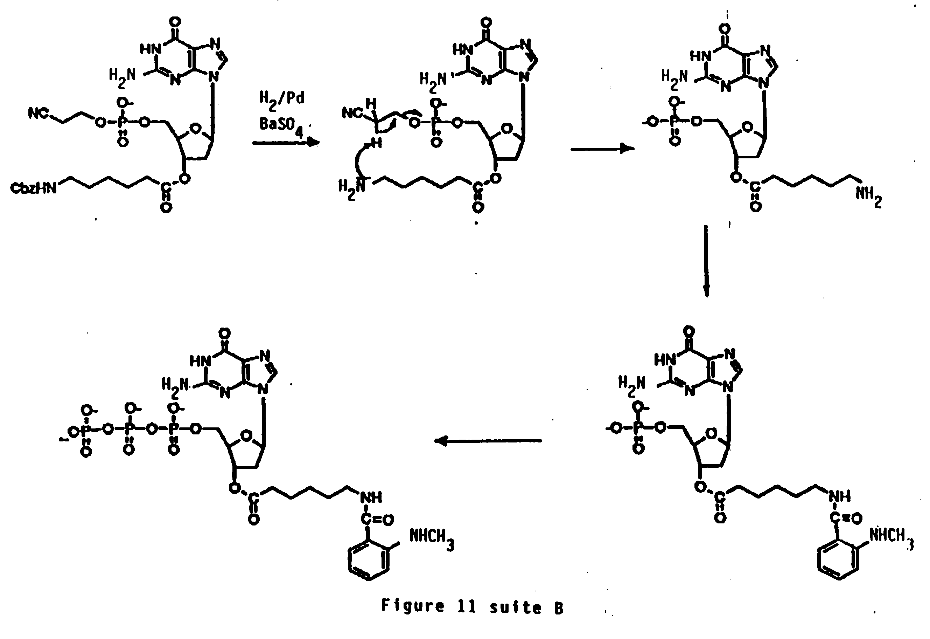

2'-désoxy-adénosine (Figure 10) :
   L'hydroxyle en 5' de la 2'-désoxy-adénosine acylée en 3' est phosphorylé par la chlorure de dibenzyle phosphate ; le phosphate et l'amine sont libérés par hydrogénolyse.
2'-désoxy-guanosine (Figure 11) :
   Le cas de la 2'-désoxy-guanosine est particulier ; en effet, le chlorure de dibenzyle phosphate ne réagit pas avec le 5'OH. Il a été phosphorylé par action du cyanoéthyle phosphate en présence de dicyclocarbodiimide. Au cours de l'hydrogénolyse du groupement benzyloxycarbonyle, le phosphodiester est transformé en phosphomonoester, par élimination intra-moléculaire du groupement cyanoéthyle. Un schéma de synthèse est représenté sur la Figure 11.

### • Incorporation des 3'-RT-dNTPs :

Environ 2 picomoles d'un 21-mer- 5'-biotinylé (5'-Bio-ATACTTTAAGGATATGTATCC-3') sont liées à des M-280 Dynabeads de la façon décrite par le fabricant et hybridées avec un excès (environ 50 pmoles) d'un oligonucléotide complémentaire présentant une queue 5' (dT)₁₀, (dC)₅, (dG)₅. ou (dA)₅. L'hybridation est faite pendant une heure à température ambiante en présence de NaCl 1M, de Tris-HCl 5mM (pH 7,5) et d'EDTA 0,5 mM. Après élimination de l'oligonucléotide non lié, les billes lavées sont mises sous suspension dans du tampon fourni par le fabricant et incubées en présence de 500 µM d'un 3'-RT-dNTP unique et d'ADN polymérase à 50°C. La réaction est terminée avec 20 mM d'EDTA, 0,01% de Triton X-100, les billes lavées et leur concentration déterminée au microscope avec une cellule hématocyter avant d'être essayées pour déterminer l'incorporation de la radioactivité (voir ci-après).

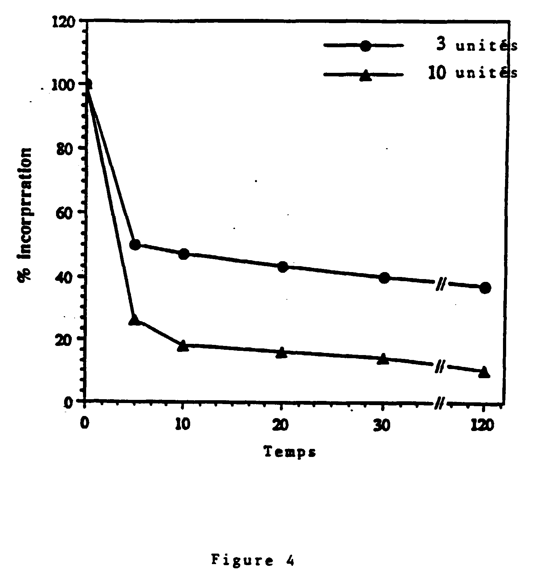

Comme ces analogues de nucléotides ne contiennent pas de groupe 3'-hydroxy, leur incorporation dans un brin d'ADN d'élongation aboutit à la terminaison de la chaîne. Ceci est montré dans la Figure 4, dans laquelle un substrat d'ADN est d'abord traité avec un 3'-RT-dATP et une ADN polymérase Taq pendant des périodes de temps différentes, avant d'être lavé et essayé pour déterminer les groupes 3'-hydroxy libres disponibles pour l'élongation de chaîne.

Dans ces conditions, environ 80% du substrat d'ADN sont bloqués pour une élongation supplémentaire dans les 10 minutes avec 10 unités d'enzyme, puis il y a une diminution lente régulière de la quantité de groupes 3'-hydroxy disponibles. Lorsque les 3'-RT-dGTP, 3'-RT-dTTP ou 3'-RT-dCTP sont testés avec leurs matrices respectives, des profils similaires sont obtenus. Si un 3'-RT-dNTP donné est incubé avec une ADN polymérase et une matrice qui ne correspond pas à son propre appariement de bases, aucune terminaison de chaîne ultérieure n'est observée, ce qui indique que le groupe 3' ne modifie pas le mécanisme de reconnaissance de l'enzyme.

Ces résultats montrent que malgré un groupe 3' relativement volumineux, ces nucléotides modifiés sont encore acceptés par l'enzyme. De nombreux analogues de nucléotides de terminaison de chaîne sont des substrats pour différentes ADN polymérases. On a montré qu'il apparaît un appariement de bases correct du substrat nucléotide avec son brin d'ADN servant de matrice et la formation de liaison phosphodiester même avec des énantiomères de β-L-ribosides [Van Draanen, N. et al. (1992) J. Biol. Chem. **267**, pp.25019-25024], ce qui indique que la liaison de la portion sucre par l'enzyme n'est probablement pas spécifique.

Les nucléotides modifiés de l'invention sont en outre acceptés comme substrats par plusieurs ADN polymérases.

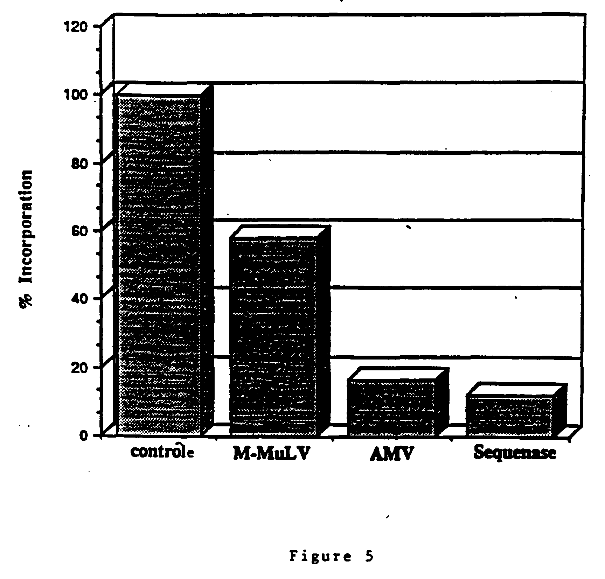

La Figure 5 montre que des analogues synthétisés ici sont des substrats pour différentes ADN polymérases, la Sequenase et la M-MuLV reverse transcriptase étant les plus et moins efficaces dans les conditions testées ici respectivement. Une ADN polymérase T7 non modifiée, une Taq polymérase et un fragment de Klenow d'ADN polymérase I commercialisé, sont aussi capables d'utiliser ces substrats. La Demanderesse n'a pas essayé d'optimiser l'incorporation avec une ADN polymérase donnée pour ce qui est de l'activité 3' → 5'-exonucléase, l'aptitude au traitement en fonction de la distributivité, ou les cinétiques de condensation.

Cependant, il apparaît clairement que le procédé ne peut être réitéré pour la base suivante que si l'enzyme condense efficacement le nucléotide en 3' et ne possède pas d'activité exo 3' → 5' qui puisse enlever le nucléotide néocondensé.

Il apparaît aussi crucial pour le procédé décrit ici que l'ADN polymérase ne possède pas d'activité estérase intrinsèque à sa chaîne polypeptidique.

Une telle activité existe chez le fragment de klenow et chez la T7 ADN polymérase, modifiée ou non.

Cette activité est indépendante du degré de purification de l'enzyme, et donc portée par la même protéine que celle possédant l'activité polymérase.

Comme cette activité estérase s'exerce en cours de polymérisation lorsqu'un nucléotide apparié correctement et estérifié en 3', cette activité estérase rend impossible le contrôle de la terminaison de chaîne lorsque les 4 3'-RT-dNTPs sont utilisés en même temps.

Cependant, la Taq ADN polymérase ne possède pas cette activité estérase sous certaines conditions de réaction comme celles décrites ici, et est donc l'enzyme de choix pour le procédé de séquençage décrit ci-après.

### • Elimination des marqueurs fluorescents et restauration de l'hydroxyle en 3' :

### a) Esters d'anthranylates :

A cette étape, un marqueur porté en 3' par le nucléotide inséré aurait deux rôles majeurs en établissant une distinction entre les quatre nucléobases possibles insérées et en bloquant un procédé ultérieur d'élongation. L'élimination du marqueur va fournir une identification indirecte de la base insérée ainsi que la régénération d'un groupe 3'-hydroxy libre disponible pour la répétition du procédé.

La nature de la liaison chimique entre la partie ribosyle et les substituants anthranyloyles est particulièrement importante. Les esters carboxyliques sont facilement hydrolysés par des quantités équivalentes d'ions hydroxyde compatibles avec la stabilité chimique des ADN monocaténaires [Nucleic Acid Chemistry, Townsend, L.B. and Tipson, S.R. eds., John Willey and Son, New-York et Jencks, W.P. (1969) Catalysis in Chemistry and Enzymology, Dover publication, Inc., New-York]. De plus, de nombreuses différentes estérases et des sérine protéases catalysent l'hydrolyse d'une large variété de ces esters [Heymann, E. et Mentlein, R. (1981) Methods Enzymol. **77**, pp. 333-344], comme le fait la protéinase K sur un 3'-anthranyloyl-2'-dAMP du type I et II décrit page 6. II pourrait être possible d'estérifier la position 3' avec un marqueur portant un bras d'espacement conçu spécifiquement pour pouvoir être facilement et efficacement éliminé, soit enzymatiquement, soit chimiquement.

Ensuite, le 3'-RT-dAMP est ajouté au groupe 3'-hydroxy libre d'une matrice d'ADN comme cela est décrit plus haut et l'ester carboxylique ensuite hydrolysé avec de l'hydroxyde de sodium 0,1 M pendant 5 minutes à 37°C (Tableau 2). Après ré-hybridation du brin complémentaire, l'ADN polymérase pourrait incorporer entre 83 et 103% de radioactivité par rapport à la même matrice qui n'a pas été bloquée en 3'. Le fait que l'on ne récupère pas toujours 100% pourrait provenir d'une réhybridation incomplète de l'oligonucléotide plus que du traitement alcalin dur. Des conditions analogues d'hydrolyse d'un ester de ribosyle ont été rapportées par d'autres [Falbriard, J.G., Posternak, T. and Sutherland, E.W. (1967) Biochim. Biophys. Acta **148**, pp.99-105 et Hiratsuka, T. (1982) J. Biol. Chem. **257**, pp. 13354-13358]. La Demanderesse n'a pas observé une notable différence d'efficacité de l'hydrolyse d'ester entre les quatre 3'-RT-dNTPs incorporés.

### b) Esters de l'acide caproïque :

La libération de l'acide caproïque substitué par le fluorophore est réalisée par action d'une enzyme, par exemple une lipase ou une hydrolase.
Afin de faciliter la déprotection du phosphate, notamment pour application à des quantités de produits plus importantes, nous nous sommes orientés vers une autre méthode qui utilise comme agent de phosphorylation (P.T. Gilham et H.G. Khorana. J.A.C.S. 1958, **80**:6212-6222), le chlorure de dibenzyle phosphate (composé B) ; figure 9). Bien qu'elle soit assez limitative (48 %) pour la synthèse, cette solution offre un avantage non négligeable. En effet, à partir du composé (11) ; on obtient le composé (12) en une seule étape d'hydrogénolyse effectuée dans des conditions douces (température ambiante et pression atmosphérique).

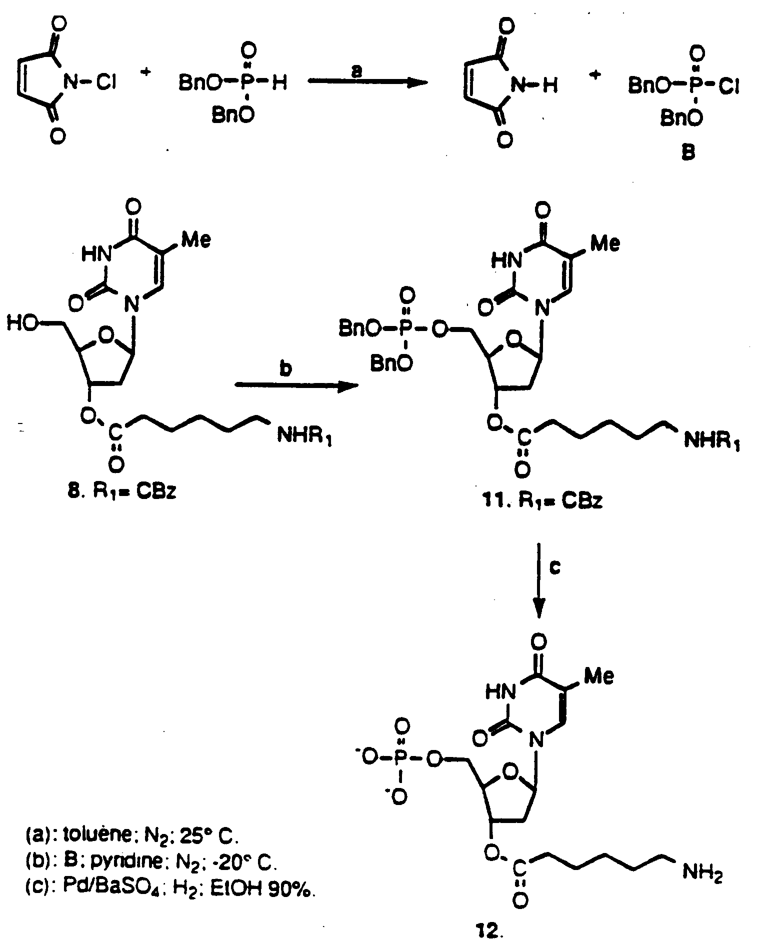

Pour la déprotection du composé (11), par hydrogénolyse (P.T. Gilham et H.G. Khorana. J.A.C.S. 1958, 80 : 6212-6222), nous avons essayé plusieurs conditions expérimentales :
- plusieurs solvants : acétate d'éthyle, éthanol, éthanol à 90 % et tétrahydrofuranne ;
- deux catalyseurs : palladium sur charbon et sur sulfate de barium.

L'éthanol à 90 %, en présence de palladium sur sulfate de barium, s'est avéré être le solvant idéal. En effet, dans celui-ci, la déprotection totale (perte des benzyles et du benzyloxycarbonyle) est quantitative et l'isolement du phosphomonoester, estérifié en 3' (composé 12) est assez aisé.

Pour la thymidine, nous avons choisi de lui assigner comme étiquette la fluorescéine (INTERBIOtech. Molecular Probes ; "1992-1994 Handbook of fluorescent probes and research chemicals". 1992, 20-41) (FITC), celle-ci répondant aux critères établis précédemment :
- étiquette non radioactive ;
- absorption (ε = 76.10⁻³ l. mol⁻¹.cm⁻¹ à λmax = 495 nm dans le DMF et à pH = 9) et émission à λₘₐₓ = 519 nm, toutes deux élevées.

Généralement, une fonction amine réagit avec une fonction isothiocyanate à un pH voisin de 11 (cette réaction a déjà été réalisée au laboratoire (S.R. Sarfati et A. Namane, Tet. Let. 1990, 31 : 2581-2584). Or, nous savons qu'à ce pH, la chaîne aliphatique greffée en 3' est hydrolysée. Pour pallier cet inconvénient, nous avons fait agir le composé (12) avec la 5-isothiocyanatefluorescéine dans un mélange pyridine/eau, à température ambiante. Ces conditions ont été décrites pour l'acide 6-amino-caproïque et la 5-isothiocyanate-fluorescéine par F.S. Wusteman et P. Gacesa (Carbohydrates Research, 1993, 241 : 237-244) (schéma ci-dessous).

Cette méthode nous a permis, à la fois de ne pas observer la formation de TMP, comme cela a lieu en milieu alcalin et de récupérer la fraction du composé (12) qui n'avait pas réagi. Par ailleurs, les difficultés que nous avons rencontrées, vis-à-vis de la solubilité du produit nous ont amenés à évaluer le rendement par chromatographie liquide à haute performance (CLHP) sur une colonne à polarité de phase inversée et à ne purifier par ce moyen que la quantité nécessaire à la caractérisation du produit.

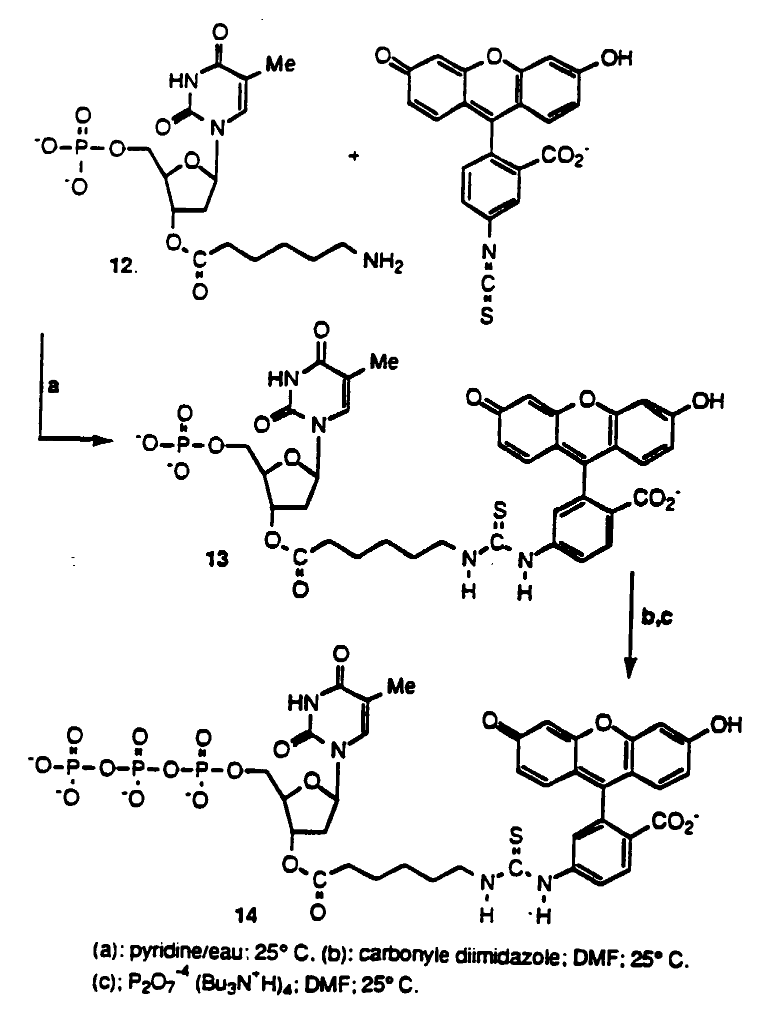

A partir du composé (13), nous avions deux possibilités pour réaliser le triphosphate de l'analogue de la thymidine (composé 14). Une première méthode consiste à préparer le phosphoromorpholidate et à déplacer la morpholine par le sel de tétra-tri-n-butylammonium de l'acide pyrophosphorique (J.G. Moffat et H.G. Khorana, J.A.C.S., 1961 83 : 649-658), une seconde à traiter le composé (13) par le carbonyldiimidazole, puis à déplacer l'imidazole par le même sel d'acide pyrophosphorique (Y. Tor et P.D. Dervan, J.A.C.S., 1993, 115 : 4461-4467 ; R. Tipson et B. Townsed, Nucleic Acid Chemistry, Part 4 (Wiley-Interscience Publication, New-York) 1991, 337-340).

Nous avons choisi cette seconde méthode, en raison des conditions basiques et de température élevée nécessitées par la première, susceptibles d'hydrolyser la chaîne aliphatique placée en 3'. Cette étape a été réalisée sur une petite échelle et sur le phosphomonoester (composé 13) non purifié, sachant que l'isolement du composé (14) nécessiterait une purification par CLHP.

Un autre mode de protection particulièrement avantageux est représenté par la synthèse des analogues triphosphates de la 2'-désoxycytidine ; l'avantage de cette méthode en est que les quatre groupements hydrogénalisables du composé (19) peuvent être éliminés en une seule étape qui est l'étape (e).

### - synthèse des analogues triphosphates de la 2'-désoxycytidine :

Partant de dC nous avons protégé, à l'aide du 1-(benzyloxycarbonyl) 3-éthylimidazolium tétrafluoro-borate, l'amine aromatique portée par la base selon les conditions de Rappoport et al. (B.E. Watkins, J.S. Kiely et H. Rappoport, J.A.C.S., 1982, 104 : 5702-5708) et la fonction alcool primaire 5' par diméthoxytritylation (M.J. Gait, Oligonucleotides synthesis a practical approach (IRL Press at Oxford University Press), 1984, 27-34 et 51 ; H. Schaller, G. Weimann, B. Lerch et H.G. Khorana, J.A.C.S. 1963, 85 : 3821-3827) (composé 16 : figure 11).

Nous avons ensuite introduit la chaîne aliphatique par action de l'anhvdride (6') sur le composé (16) (D.H. Rammler et H.G. Khorana, J.A.C.S. 1963, 85 : 1997-2002). Cette synthèse du composé (17) a aussi été réalisée par acylation "directe" du composé (6) sur le composé (16), en présence de 1,8-dicyclohexylcarbodiimide (DCCI), dans des conditions stoechiométriques et avec un rendement équivalent (85 %).

Après déprotection de la fonction hydroxyle primaire 5' du composé (17), effectuée en milieu acide comme décrit (M.J. Gait, Oligonucleotides synthesis a practical approach (IRL Press at Oxford University Press), 1984, 27-34 et 51; H. Schaller, G. Weimann, B. Lerch et H.G. Khorana, J.A.C.S. 1963, 85 : 3821-3827), nous avons phosphorylé le composé (18) par action du chlorure de dibenzyle phosphate (P.T. Gilham et H.G. Khorana. J.A.C.S. 1958, 80 : 6212-6222) (composé B ; figure 9). Cette étape, assez limitative (48 %), a permis d'obtenir le composé (19) attendu. Sa déprotection, par hydrogénolyse (P.T. Gilham et H.G. Khorana. J.A.C.S. 1958, 80 : 6212-6222 ; B.E. Watkins, J.S. Kiely et H. Rappoport, J.A.C.S., 1982, 104 : 5702-5708 ; J.H. Rigby, T.L. Moore et S. Rege, J.O.C. 1986, 51 : 2400-2402), a été réalisée dans des conditions identiques à celles employées pour l'analogue de la thymidine et avec un rendement élevé (78 %).

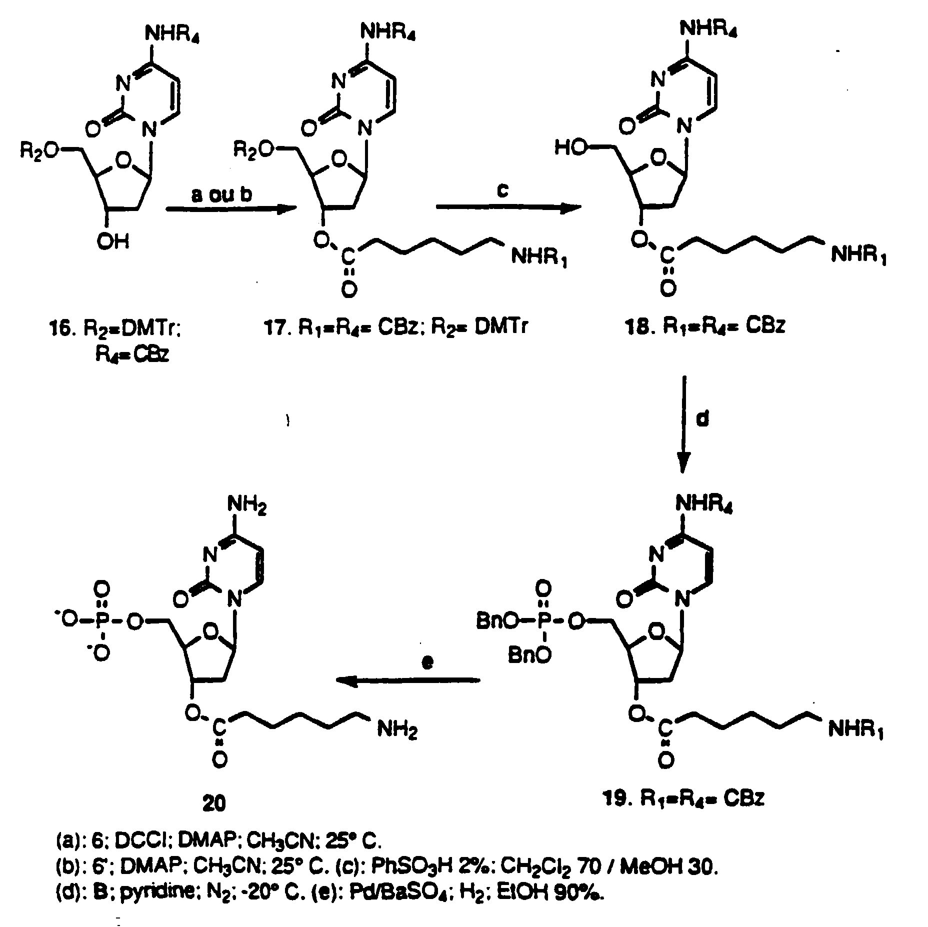

Nous avons ensuite choisi dans un premier temps comme étiquette de la 2'-désoxycytidine (dC), l'acide N-méthylisatoïque (figure 13) pour plusieurs raisons :
- l'action de l'anhydride N-méthylisatoïque n'affecte pas l'amine aromatique portée par la base pyrimidique de dC (B. Canard et R.S. Sarfati, 1994 (Gene, sous presse) ;
- cette étiquette, excitée à λₘₐₓ = 338 nm, émet à une longueur d'onde λₘₐₓ = 416,5 nm, distincte de celle de la fluorescéine (λₘₐₓ d'émission = 519 nm) ;
- la synthèse du composé (21) nous permettrait de vérifier rapidement si notre stratégie pour obtenir le triphosphate, à partir du monophosphate étiqueté en 3' par l'intermédiaire de la chaîne aliphatique (composé 6), était bonne.

A partir du composé (21) synthétisé dans les mêmes conditions expérimentales que pour l'analogue de la thymidine avec la 5-isothiocyanatefluorescéine (F.S. Wusteman et P. Gacesa Carbohydrates Research, 1993, 241 : 237-244), nous avons préparé le triphosphate, par traitement successif du composé (21) avec le carbonyldiimidazole et avec l'acide pyrophosphorique, sel de tétra-tri-n-butylammonium (Y. Tor et P.D. Dervan, J.A.C.S., 1993, 115 : 4461-4467 ; R.Tipson et B. Townsed, Nucleic Acid Chemistry, Part 4 (Wiley-Interscience Publication, New-York) 1991, 337-340). Le composé (22), ainsi obtenu, a été purifié par chromatographie et caractérisé par résonance magnétique nucléaire du phosphore.

Ces bons résultats ont incité à étiquetter, également, le composé (20) avec la rhodamine (INTERBIOtech. Molecular Probes ; "1992-1994 Handbook of fluorescent probes and research chemicals". 1992, 20-41). En effet, cette dernière fluoresce beaucoup plus fortement que l'acide N-méthylisatoïque et à une longueur d'onde, toujours distincte de celle de la fluorescéine, λₘₐₓ d'émission = 596 nm.

En raison de la faible solubilité du composé (23), seulement, une fraction a été purifiée par CLHP pour le caractériser.

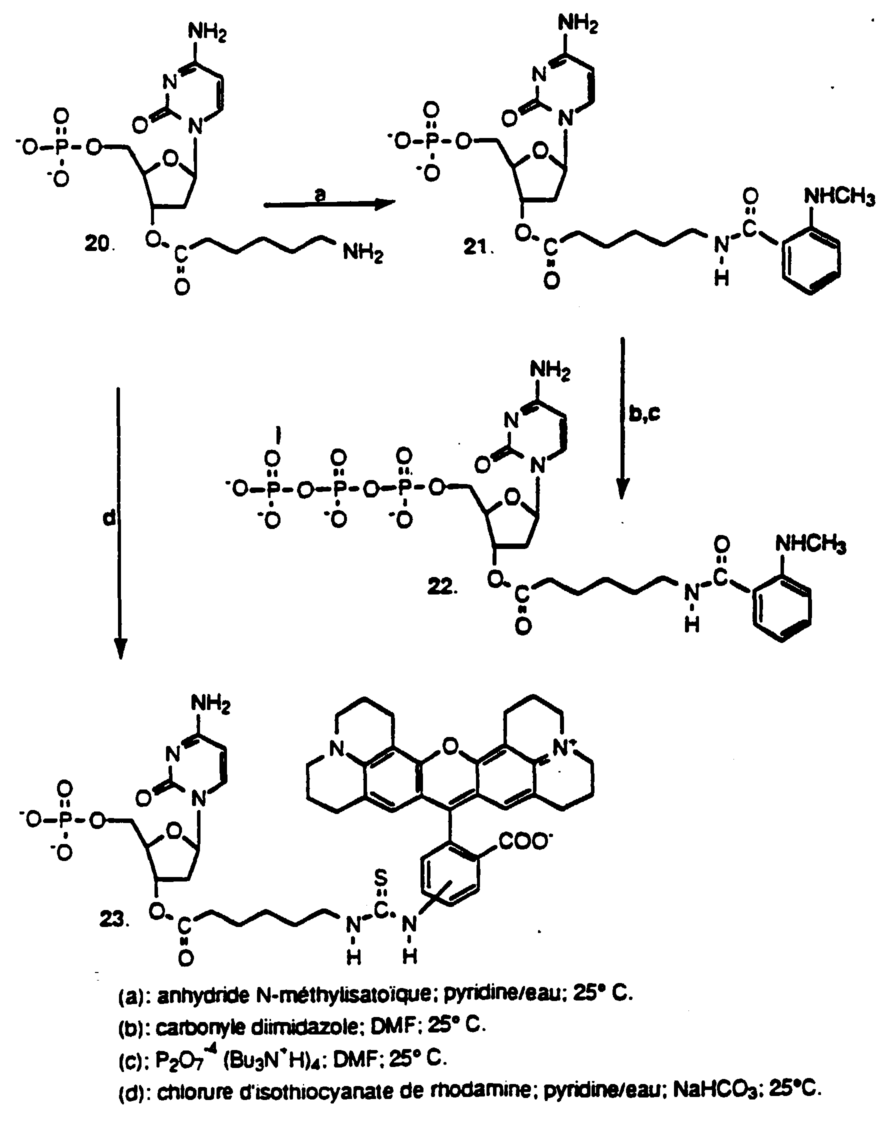

### c) Esters de l'acide 5-amino-2, 5-didésoxy D-ribonique :

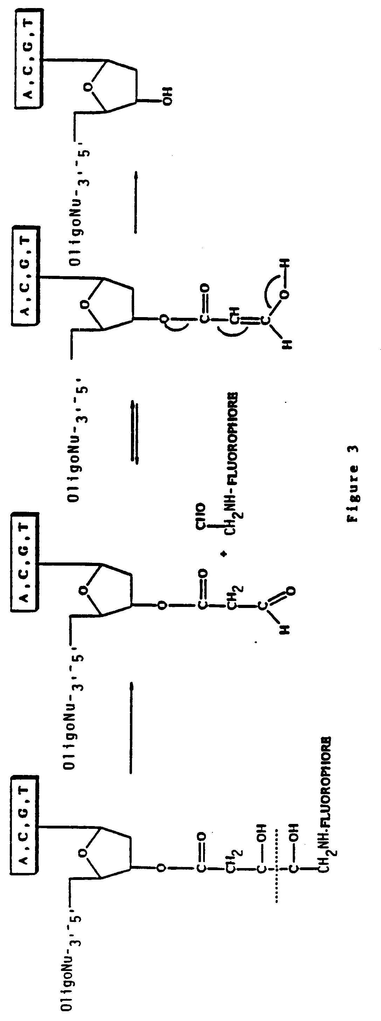

Le schéma de la Figure 3 indique comment la libération et la restauration du groupe hydroxyle sont réalisées : le diol vicinal est oxydé par le périodate, cette oxydation conduisant, d'une part, à un aldéhyde substitué par le fluorophore dont l'absorption et l'émission de fluorescence peuvent être mesurées par les méthodes décrites plus haut, et, d'autre part, à un oligonucléotide substitué par un aldéhyde, lequel, après énolisation, est éliminé par β-élimination, libérant ainsi l'hydroxyle en 3'.

L'utilisation d'une oxydation par le periodate est compatible avec une nouvelle élongation sans rehydridation de l'amorce puisque ces conditions ne dénaturent pas l'ADN double chaîne tel l'amorce hybridée sur la matrice à séquencer.

L'ensemble des opérations peut être ainsi réitéré jusqu'à ce que la séquence d'intérêt soit entièrement séquencée.

Le schéma de la figure 9 illustre bien les avantages de ce type de composés ; en effet, en synthèse oligonucléotidique, les sucres sont souvent temporairement protégés sous la forme d'esters de l'acide lévulinique, les lévulinates (J.H. Vanboom et al., Tetrahedron Lett. 4875 (1976)). Entre autres intérêts de ces esters, la déprotection des alcools est quantitative en 1 minute, à pH neutre et à température ambiante. Dans ces conditions, les oligonucléotides sont stables.

Les nucléotides acylés en 3' par l'acide 6-amino-2, 3, 6-tridésoxy-D-gluconique oxydés par le periodate, libèrent quantitativement le fluorochrome et un ester qui ne diffère de l'ester lévulinique que par le remplacement d'un méthyle par un hydrogène (une cétone par un aldéhyde).

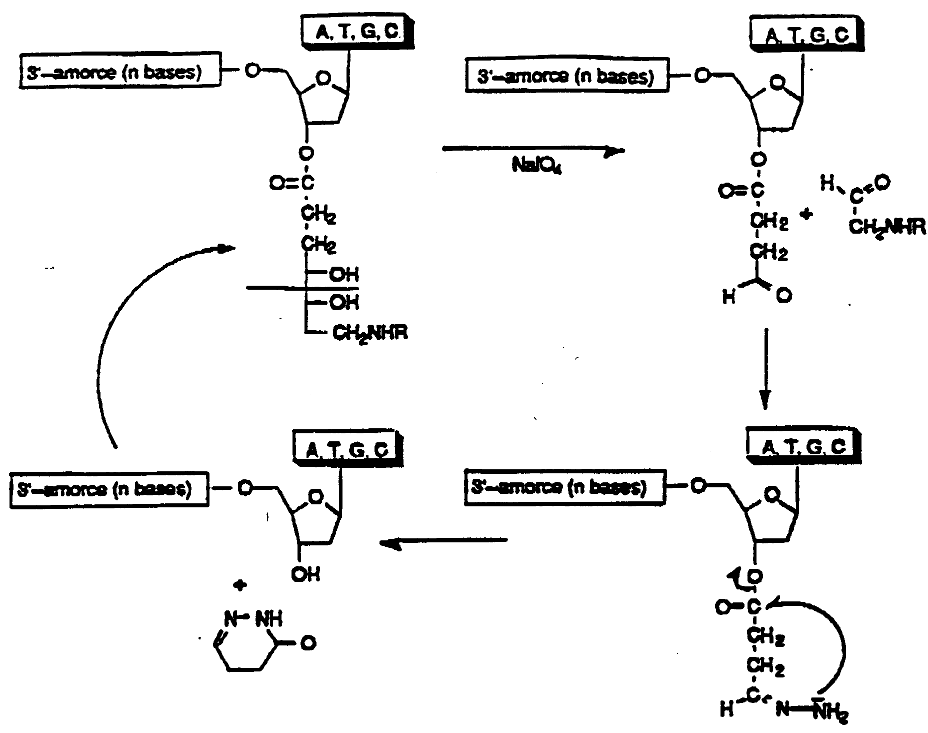

Les alcools sont régénérés par le mécanisme suivant : dans un mélange hydrazine-pyridine-acide acétique-eau, à pH neutre, la fonction cétone est transformée en hydrazone, le doublet libre sur l'azote attaque le carbonyle et l'alcool est libéré.

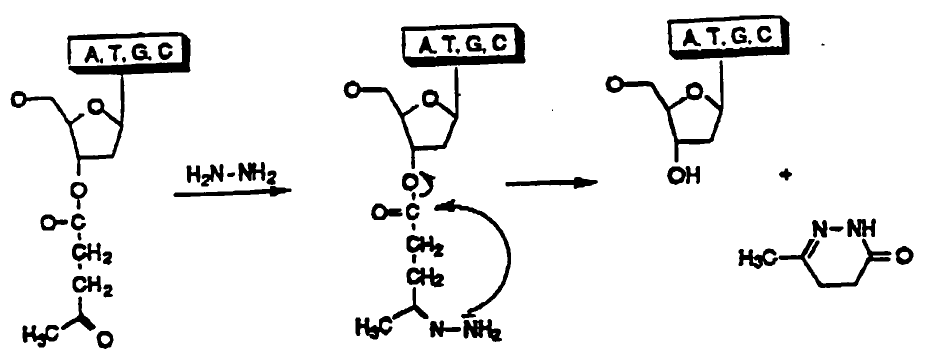

Les hydrazones se forment plus facilement à partir des aldhéhydes que des cétones, la restauration du 3'-OH devrait donc être plus rapide à partir d'un aldéhyde.

Ce type de composés présente donc de nombreux avantages : ils sont déprotégeables rapidement à température ambiante, à pH neutre et surtout quantitativement.

### - Exemple d'un séquençage de nucléotide le long du gène rpoB d'une souche résistante à la rifampicine de Mycobacterium leprae.

La démonstration que le procédé décrit ci-dessus peut être utilisé pour identifier les séquences de nucléotides dans un mélange complexe d'acides nucléiques simple chaîne dans un tube ou dans une colonne, laquelle méthode évite l'étape d'électrophorèse en gel de polyacrylamide, est illustrée par l'exemple ci-dessous.
1) Echantillon d'ADN et cible de séquençage :
   Dans cet exemple, la séquence nucléotidique destinée à être analysée est une partie du gène de la sous-unité β de l'ARN polymérase de Mycobacterium leprae (rpoB).
   Un changement d'un amino acide dans le codon 425 dans le gène confère à la bactérie une résistance à la rifampicine. Des mutations aux positions 1, 2 ou 3 dans le codon sauvage ont été déjà décrites et qui remplacent une serine par une leucine, une phénylalanine ou méthionine [Honoré N. et Cole S.T. (1993), Antimicrobiol. Agence and Chemotherapy 37, pp. 414-418].
   Le matériel biologique de départ est obtenu comme décrit dans la référence ci-dessus mentionnée ; brièvement, des cellules de M. Leprae résistantes à la rifampicine sont extraites de la plante de pied de souris et sont lysées par une technique de congélation/ébullition et les échantillons soumis à une amplification par PCR en utilisant les amorces 1 et 2 spécifiques du gène rpoB. Le produit d'amplification est ensuite analysé par électrophorèse en gel d'agarose et utilisé comme un échantillon d'ADN de départ dans le présent exemple.
2) Amorce oligonucléotidique :
   L'amorce 1 et le Brpo B22 de la référence ci-dessus. C'est une amorce 5' biotinylée avec la séquence suivante :
   5'-CAGGACGTCGAGGCGATCAC-3'.
   L'amorce 2 est choisie dans la séquence adjacente au nucléotide à analyser dans le codon 425 du gène rpoB.
   Il est constitué de 21 nucléotides avec la séquence suivante :
   5'-CAAACCACCCGGGCCCAGCGC-3'
   laquelle séquence correspond au type sauvage du gène rpoB de M. Leprae en aval du codon 425.
   L'amorce 3 est l'amorce en "épingle à cheveux" décrite dans le texte ci-dessus et dans la figure 7.
   Son extrémité 5' est phosphorylée et a la séquence nucléotidique suivante :
   5'-GGGCGGCGGGGCTTTATTTGCCCCGCCGCCCCAAACCACCCGGGCCCAGCGC-3'.
   Ces amorces ont été obtenues sous une forme purifiée auprès de la Société GENSET corp. (Paris, France).
3) Préparation des matrices pour le séquençage :
   Les amorces 1 et 2 sont utilisées exactement comme décrit dans la référence ci-dessus (Honoré et al.) pour produire un fragment d'amplification par PCR utilisable pour le séquençage du codon 425 d'un échantillon inconnu de M. Leprae dans un volume de réaction final de 100 µl.
   Un aliquot (10 µl) est analysé par électrophorèse en gel d'agarose pour vérifier que l'amplification PCR est correcte.
   100 µl du produit d'amplification PCR sont ensuite absorbés sur des billes magnétiques (M280 Dynabeads, streptavidine, Dynal corp., Norvège) recouvertes de streptavidine et prélavées et les chaînes d'ADN non biotinylées et dénaturées et élevées de sa chaîne complémentaire qui reste liée aux billes en utilisant 0,1 M de NaOH tel que décrit par le fabricant.
   Après plusieurs lavages, la matrice d'ADN simple chaîne liée au support est dans une forme purifiée sans interférer avec des amorces ou des nucléotides.
4) Ligation de l'amorce en "épingle à cheveux" n° 3 :
   La matrice liée au support est resuspendue dans un tampon de ligation (40 mM Tris pH 7.5, 10 mM MgCl₂, 10 mM DTT, 0,5 mM ATP), 5 nm de l'amorce n° 3 en "épingle à cheveux" sont ajoutés au mélange, le tube est chauffé 2 minutes à 69°C et 30 minutes à 37°C.
   Après l'addition de 40 unités d'ADN ligase T4 (Boehringer corp., Allemagne) le mélange est incubé pendant 3 h à température ambiante avec agitation de temps en temps.
   Le produit ainsi lié attaché aux billes et lavé plusieurs fois comme cela est décrit par le fabricant, le dernier lavage étant dans un tampon TE (10 mM Tris pH 8, 1 mM EDTA) et enfin resuspendu dans 120 µl d'eau distillée stérile.
5) Réaction de séquençage :
   a) Incorporation d'un 3' RT-dNMP à l'extrémité 3' OH de l'amorce 3 en "épingle à cheveux" lié (matrice de séquençage) :
      La matrice de séquençage est maintenant étendue par l'amorce en "épingle à cheveux" qui donne une extrémité 3' OH bloquée (voir Figure 7). La solution suivante (solution d'élongation) est ajoutée à la matrice liée aux billes :
      - 10 µl d'un tampon concentré 10 fois (donné par le fabricant),
      - 10 µl de glycérol,
      - 50 µl de 3' RT-dNTP à la concentration de 2 mM chacun : 3'-anthranyloyle dATP, 3'-N-méthyl-anthranyloyle dGTP, 3'-5-méthyl-anthranyloyle dCTP, 3'-6-méthyl-anthranyloyle dTTP,
      - 10 µl de Taq polymérase (5 unités/µl, Boehringer corp., Allemagne).

      Le tube est incubé à 50°C pendant 30 minutes avec une agitation douce occasionnelle et ensuite transféré sur un séparateur magnétique pour éliminer les nucléotides non incorporés : 3 lavages avec 10 mM de Tris pH 7.5, 1 mM EDTA, 2 M NaCl, 0,02 % Triton X-100, suivis par 2 lavages avec 0,02 % de Triton X-100 et 1 lavage avec de l'eau distillée stérile.
   b) Elimination de la protection en 3' :
      L'eau distillée stérile est enlevée et 30 µl de NaOH 0,1 M sont ajoutés directement sur les billes.
      L'incubation est ensuite réalisée à 50 °C pendant 10 minutes. L'échantillon est ensuite magnétisé, le surnageant collecté, 30 µl de 0,1 M NaOH sont ajoutés de nouveau sur les billes et incubés à 50°C pendant 10 minutes, puis après une séparation magnétique, le deuxième surnageant est mélangé avec le premier avant l'analyse spectrofluorométrique.
      Les billes sont immédiatement lavées exactement comme ci-dessus après l'étape d'incorporation et 120 µl de la suspension de billes est maintenant prête pour une nouvelle réaction d'extension.
   c) Identification du marquage en 3' correspondant au nucléotide incorporé :
      6,6 µl de Tris 0,5 M pH 8 sont ajoutés aux surnageants rassemblés et le mélange est soumis à une analyse spectrofluorométrique comme décrit plus haut en utilisant un spectrofluoromètre Perkin-Elmer L50B et la cellule de détection spectrofluorométrique HPLC correspondante.

   La calibration est réalisée en utilisant des solutions témoins contenant 0,1 M NaOH et 50 mM Tris pH 8.
   Des spectres individuels correspondant aux dérivés dépourvus d'anthranylate sont réalisés dans le même tampon et leur émission maximale déterminée.
   Ensuite, leurs spectres d'excitation sont déterminés comme décrit plus haut.
   De cette manière, chaque marqueur est défini avec deux longueurs d'ondes spécifiques, à savoir le maximum en nanomètres pour l'excitation et pour l'émission.
   Après avoir chargé l'échantillon dans la cellule de détection, l'excitation est réalisée à 310 nm et le spectre d'émission est mesuré entre 340 et 500 nm.
   Le spectre est corrigé de tout effet venant du tampon en soustrayant le spectre du témoin, la courbe est lissée et les longueurs d'ondes correspondant au maximum décrit ci-dessus sont déterminées en utilisant un programme d'ordinateur fourni par le fabricant.
   Les surnageants soumis à l'analyse spectrofluorométrique donnent comme résultats 317 nm et 409 nm pour les maximum d'excitation et d'émission respectivement.
   Ceci identifie de façon non ambiguë un résidu dC incorporé à l'extrémité 3' de la matrice de séquençage.
6) Réitération du procédé : séquençage du codon 425 du gène rpoB de M. leprae d'un isolat résistant à la rifampicine :
   Les 120 µl de suspension de billes sont maintenant soumis de nouveau aux étapes 5 a), b), c) de la réaction de séquençage, afin d'identifier le nucléotide suivant du codon 425 de l'isolat résistant à la rifampicine de M. leprae.
   Dans ce cas, les maximum d'excitation et d'émission sont de 315 nm et de 397 nm respectivement et ceci identifie un résidu dA incorporé après le dC de la précédente réaction.
   Le cycle de séquençage complet suivant (étape 5 a), b) et c)) identifie les longueurs d'ondes d'excitation et d'émission à 289 nm et 403 nm respectivement, ce qui est typique d'un résidu dT incorporé à l'extrémité 3' de la matrice de séquençage.

Ainsi, des expériences nous concluons que la séquence nucléotidique du codon 425 du gène rpoB de l'échantillon analysé est ATG, ce qui code pour une méthionine au lieu d'une sérine, la dernière étant caractéristique du phénotype sauvage.

Du fait que cette sorte de mutation a été décrite dans des isolats de type rpoB3 (voir Honoré N. et al.), les expériences décrites ci-dessus donnent les bases moléculaires de la résistance à la rifampicine dans le gène de l'isolat de M. Leprae.

## Revendications

1. Esters de désoxyribonucléotides 5' triphosphates (dNTP) diguanosine cytosine thymine ou ribonucléotides 5' triphosphates (NTP), caractérisés par le fait qu'ils sont des anthranylates répondant à la formule générale suivante : 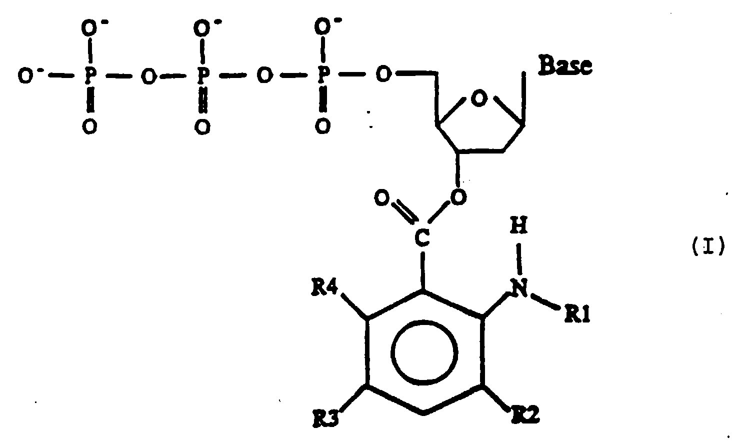
dans laquelle R1, R2, R3 ou R4 sont soit un atome d'hydrogène (H), soit un groupe méthyle (CH₃), soit un groupe autre pourvu que les propriétés du résidu soient conservées,
dans laquelle quand la base est l'adénine, au moins un des radicaux R1, R2, R3 ou R4 est différent de H,
et dans laquelle l'hydroxyle en 3' peut être restauré par libération du résidu anthranylique en milieu basique ou par action d'une enzyme, par exemple une lipase ou une hydrolase.

2. Esters selon la revendication 1, caractérisés par les substitutions suivantes :
R1=CH₃, R2=R3=R4=H dans le N-méthyl 3' anthranylate-dGTP
R2=CH₃, R1=R3=R4=H dans le 3-méthyl 3' anthranylate-dTTP
R3=CH3, R1=R2=R4=H dans le 5-méthyl 3' anthranylate-d-CTP

3. Utilisation des esters selon l'une des revendications 1 ou 2, dans un procédé de séquençage d'une chaîne polynucléotidique comprenant les étapes de :
a) construction et utilisation d'une amorce,
b) addition à partir de cette amorce, par une acide nucléique polymérase, d'un nucléotide triphosphate estérifié en 3'OH de telle façon que son intégration bloque toute élongation ultérieure et que l'ester porte un marquage spécifique de chacune des quatre bases constituant ces nucléotides modifiés,
c) restauration de la fonction 3'OH par hydrolyse chimique ou enzymatique de l'ester formé,
d) caractérisation de l'hydrolysat formé caractéristique d'un nucléotide donné,
e) réitération des étapes a), b), c) et d) pour caractériser le nucléotide suivant.

4. Utilisation selon la revendication 3, caractérisé en ce que l'amorce est une amorce en "épingle à cheveux" phosphorylée en 5' et présentant une partie de sa séquence en 3' identique à celle d'une amorce utilisée en PCR pour produire la matrice d'ADN à séquencer et qui, après ligation à la matrice d'ADN à séquencer, cette amorce est compatible avec une déprotection en milieu basique.

5. Utilisation selon la revendication 3, caractérisé en ce que l'ester est porteur d'un composé détectable en fluorescence, les caractéristiques d'excitation et d'émission dudit composé étant caractéristiques pour chacun des quatre nucléotides.

6. Utilisation des esters selon l'une des revendications 1 ou 2 à la détection de mutations ponctuelles dans une séquence d'acides nucléiques, ou de variants impliquant entre 2 et 20 nucléotides.

7. Utilisation des esters selon l'une des revendications 1 ou 2 à la recherche d'une séquence particulière dans un mélange complexe d'acides nucléiques.

8. Trousse de diagnostic de la présence dans un échantillon d'une séquence d'acides nucléiques que l'on souhaite analyser et comprenant :
- une amorce de séquençage,
- 4 désoxyribonucléotides estérifiés en 3' de façon réversible conformément à la revendication 1,
- éventuellement une phase solide pour immobiliser l'acide nucléique à analyser ou l'amorce,
- une acide nucléique polymérase choisie en fonction de l'amorce, et préférentiellement sans activité 3' ---> 5' exonucléasique, préférentiellement l'ADN Taq polymérase.

9. Composé de formule:
